# EUROPÄISCHE PATENTANMELDUNG

(11) **EP 3 679 958 A1**
(43) Veröffentlichungstag der Anmeldung: **15.07.2020**
(21) Anmeldenummer: 19151005.6
(22) Anmeldetag: 09.01.2019
(51) Int. Cl.: A61M 1/00

(54) **SAUGPUMPE MIT OPTISCHEM STATUSINDIKATOR**

(71) Anmelder: Medela Holding AG, 6340 Baar (CH)
(72) Erfinder: Bächler, Cornel, 6038 Honau (CH); Imhof, Thomas, 4805 Brittnau (CH); Ehlert, Hilmar, 6052 Hergiswil NW (CH)
(74) Vertreter: Grünecker Patent- und Rechtsanwälte PartG mbB

(57) **Zusammenfassung**

Die Erfindung betrifft in einer Variante eine Saugpumpe (1) zum Absaugen von Körperflüssigkeit mit einem optischen Statusindikator (5) zur Anzeige eines Lichtsignals in Abhängigkeit vom Betriebszustand der Saugpumpe (1), dadurch gekennzeichnet, dass der optische Statusindikator (5) auf mindestens zwei Punkten der äußeren Oberfläche der Saugpumpe (1) angeordnet ist, deren Flächennormalen in einem Winkel von mindestens 5° zueinanderstehen. In einer zweiten Variante betrifft die Erfindung eine Saugpumpe (1) zum Absaugen von Körperflüssigkeit mit einem optischen Statusindikator (5) zur Anzeige eines Lichtsignals in Abhängigkeit vom Betriebszustand der Saugpumpe (1), wobei die Saugpumpe (1) auf ihrer Unterseite mindestens einen Standfuß (13) zur Auflage auf einer Auflagefläche aufweist, dadurch gekennzeichnet, dass der Statusindikator (5) auf der Unterseite der Saugpumpe (1) angeordnet ist, so dass das Lichtsignal in Richtung der Auflagefläche abgegeben wird. In einer dritten Variante betrifft die Erfindung eine Saugpumpe (1) zum Absaugen von Körperflüssigkeit mit einem optischen Statusindikator (5) zur Anzeige eines Lichtsignals in Abhängigkeit von Betriebszustand der Saugpumpe (1), dadurch gekennzeichnet, dass der Statusindikator (5) mindestens 5% der Oberfläche der Saugpumpe (1) einnimmt.

## Beschreibung

### Technisches Gebiet

Die vorliegende Erfindung betrifft eine Saugpumpe, insbesondere eine Brustpumpe zum Abpumpen von menschlicher Muttermilch oder eine Drainagepumpe zum Absaugen von Körperflüssigkeiten, beispielsweise für die Thoraxdrainage oder für die Wunddrainage.

### Stand der Technik

Saugpumpen der eingangs erwähnten Art werden in verschiedenen Bereichen, insbesondere als medizinische Saugpumpen zum Absaugen von Körperflüssigkeiten eingesetzt. Beispielsweise werden sie als Brustpumpen zum Abpumpen von menschlicher Muttermilch, Wund- oder Thoraxdrainage oder zum Absaugen von Körperflüssigkeiten verwendet.

Medizinische Saugpumpen weisen üblicherweise eine Vakuumpumpe, einen oder mehrere Fluidsammelbehälter und eine Schlauchverbindung zwischen Patient und Fluidsammelbehälter auf. Die Schlauchverbindung ist üblicherweise mit einer Fluidsammelvorrichtung, beispielsweise einer Brusthaube oder einem Vakuumverband, verbunden, die auf dem Körper des Patienten aufliegt. Mittels der Vakuumpumpe wird ein Unterdruck innerhalb der Fluidsammelvorrichtung erzeugt, wodurch Flüssigkeit vom Körper durch die Schlauchverbindung in den Fluidsammelbehälter gesaugt wird. Eine beispielhafte medizinische Saugpumpe ist in WO 2017/157691 A1 offenbart.

Um einen ordnungsgemäßen Betrieb der Saugpumpe zu gewährleisten, müssen die Betriebsparameter, wie insbesondere der angelegte Unterdruck und der Füllstand des Fluidsammelbehälters, laufend überwacht werden. Beispielsweise beschreibt WO 2015/197462 A1 eine medizinische Saugpumpe mit einem Füllstandsensor zur Überwachung des Füllstands im Fluidsammelbehälter.

Üblicherweise weisen bekannte Saugpumpen eine Anzeige auf, von der sich der aktuelle Betriebszustand der Saugpumpe ablesen lässt. Diese Anzeige lässt sich jedoch in der Regel nur aus nächster Nähe und aus einem bestimmten Blickwinkel ablesen. Dies erhöht einerseits den Arbeitsaufwand beim Überwachen einer Vielzahl von Saugpumpen und hat außerdem den Nachteil, dass Pflegepersonal auch in sensiblen Bereichen wie einer Intensiv- oder Isolierstation mitunter nah an den Patienten herantreten muss.

### Darstellung der Erfindung

Es ist eine Aufgabe der Erfindung, eine Saugpumpe bereitzustellen, deren Betriebszustand sich aus der Entfernung und aus mehreren Blickwinkeln einfach ablesen lässt.

Diese Aufgabe wird durch eine Saugpumpe mit den Merkmalen von Anspruch 1 oder mit den Merkmalen von Anspruch 2 gelöst.

Bei der erfindungsgemäßen Saugpumpe handelt es sich um eine Pumpe zum Absaugen von Körperflüssigkeiten, wie beispielsweise eine Brustpumpe zum Abpumpen von menschlicher Muttermilch oder eine Drainagepumpe für die Wund- oder Thoraxdrainage. Eine gattungsgemäße Saugpumpe ist beispielsweise in WO 2015/197462 A1 und WO 2017/157691 A1 offenbart. In einer besonders bevorzugten Ausführungsform handelt es sich um eine Drainagepumpe für die Wund- oder Thoraxdrainage.

Die erfindungsgemäße Saugpumpe weist einen optischen Statusindikator zur Anzeige eines Lichtsignals in Abhängigkeit des Betriebszustands der Saugpumpe auf.

Dieser Statusindikator ist gemäß einer ersten Variante der Erfindung dadurch gekennzeichnet, dass er auf mindestens zwei Punkten der äußeren Oberfläche der Saugpumpe angeordnet ist, deren Flächennormalen in einem Winkel von mindestens 5° zueinanderstehen. Als Flächennormale wird hierbei ein Vektor definiert, der senkrecht auf dem jeweiligen Punkt der äußeren Oberfläche steht und von der Saugpumpe aus gesehen nach Außen gerichtet ist.

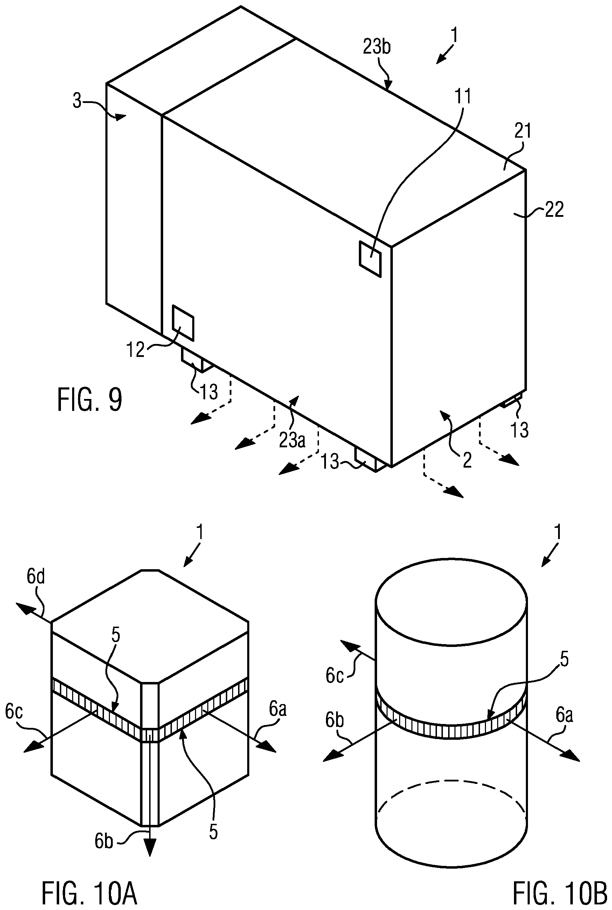

Zur Veranschaulichung der erfindungsgemäßen Anordnung des Statusindikators sei auf die Figuren 10A und 10B verwiesen.

Fig. 10A zeigt schematisch eine Saugpumpe 1 mit einer achteckigen Grundfläche und acht, jeweils in einem Winkel von 45° zueinanderstehenden jeweils ebenen Seitenflächen. Ein Statusindikator 5 ist in Form eines durchgängigen Lichtbands auf sämtlichen Seitenflächen angeordnet. Damit ist dieser Statusindikator unter anderem auf vier beispielhaft ausgewählten Punkten der äußeren Oberfläche der Saugpumpe angeordnet, deren Flächennormalen 6a-d eingezeichnet sind. Die Flächennormalen 6a und 6b stehen in einem Winkel von 45° zueinander, die Flächennormalen 6a und 6c in einem Winkel von 90° und die Flächennormalen 6a und 6d in einem Winkel von 180°. Maßgeblich ist jeweils der zwischen den nach außen zeigenden Vektoren eingeschlossene Winkel.

Fig. 10B zeigt schematisch eine Saugpumpe 1 mit kreisförmiger Grundfläche und einer zylindrischen Grundform. Ein Statusindikator 5 ist in Form eines durchgängigen Lichtbands entlang des gesamten Außenumfangs der Saugpumpe angeordnet. Damit ist dieser Statusindikator unter anderem auf drei beispielhaft ausgewählten Punkten der äußeren Oberfläche der Saugpumpe angeordnet, deren Flächennormalen 6a-c eingezeichnet sind. Die Flächennormalen 6a und 6b stehen in einem Winkel von 90° zueinander und die Flächennormalen 6a und 6c in einem Winkel von 180°.

Im Gegensatz zu der erfindungsgemäßen Saugpumpe verfügen herkömmliche Saugpumpen zur Anzeige des Betriebszustands über Displays oder beleuchtete Schalter, welche nur auf einer einzigen ebenen Fläche des Pumpengehäuses und somit nur auf Punkten der äußeren Oberfläche angeordnet sind, deren Flächennormalen parallel und damit in einem Winkel von weniger als 5° zueinanderstehen.

Durch die erfindungsgemäße Anordnung des Statusindikators auf mindestens zwei Punkten der äußeren Oberfläche der Saugpumpe wird das Lichtsignal in einem breiten Blickwinkelbereich abgestrahlt und ist somit auch aus größerer Entfernung gut zu erkennen. Auf diese Weise kann der Betriebszustand der Saugpumpe auf einfache Weise überprüft werden.

Der Statusindikator ist erfindungsgemäß auf mindestens zwei Punkten der äußeren Oberfläche der Saugpumpe angeordnet, deren Flächennormalen in einem Winkel von mindestens 5°, bevorzugt mindestens eines ganzzahligen Vielfachen von 5°, bis maximal 180° zueinanderstehen, wobei der jeweils kleinere, zwischen zwei Flächennormalen eingeschlossene Winkel in Betracht zu ziehen ist. Beispielsweise stehen die Flächennormalen in einem Winkel von mindestens 10°, weiter bevorzugt mindestens 15°, mindestens 20°, mindestens 25°, mindestens 30°, mindestens 35°, mindestens 40°, mindestens 45°, mindestens 50°, mindestens 60°, mindestens 70°, mindestens 80°, mindestens 90°, mindestens 110°, mindestens 130°, mindestens 150° oder mindestens 170° zueinander. Ein besonders vorteilhafter Blickwinkel wird dadurch erzielt, dass der Statusindikator auf mindestens zwei Punkten der äußeren Oberfläche der Saugpumpe angeordnet ist, deren Flächennormalen in einem Winkel von mindestens 45°, bevorzugt mindestens 90°, besonders bevorzugt in einem Winkel von 180° zueinanderstehen. Des Weiteren ist besonders bevorzugt, wenn der Statusindikator auf mindestens drei Punkten der äußeren Oberfläche der Saugpumpe angeordnet ist, deren Flächennormalen jeweils in einem Winkel von mindestens 45°, bevorzugt mindestens 90° zueinanderstehen.

Vorzugsweise sind die Punkte, auf denen der Statusindikator angeordnet ist, Teil des Gehäuses der Saugpumpe. Der Statusindikator kann dabei sowohl auf der Vorderseite - also der dem Nutzer zugewandten Oberfläche des Gehäuses - auf der Rückseite, auf der Oberseite, auf der Unterseite oder auf den Seitenflächen des Gehäuses angeordnet sein. Das Gehäuse bildet die äußere Umfassung der Saugpumpe. Der Statusindikator selbst wird im Rahmen der vorliegenden Erfindung nicht als Teil der äußeren Oberfläche der Saugpumpe und damit nicht als Teil des Pumpengehäuses betrachtet, auf dem zumindest einer der zwei Punkte angeordnet ist. Der Statusindikator muss nicht notwendigerweise bündig mit der Oberfläche abschließen, sondern kann auch räumlich aus der Oberfläche herausragen, beispielsweise in Form eines beleuchteten Schalters oder eines hervorstehenden Leuchtelements.

Das Pumpengehäuse ist beispielsweise aus Kunststoff oder Aluminium gefertigt. Das Pumpengehäuse ist insbesondere so ausgebildet, dass die freiliegenden Oberflächen des Gehäuses einen allseits abdichtenden Abschluss zu den innerhalb des Pumpengehäuses angeordneten Komponenten ausbilden. Üblicherweise ragen allein Schläuche und Schalter aus dem Pumpengehäuse heraus, wenn diese Teil der Pumpe sind. Gegebenenfalls ragen auch lediglich Schlauchstutzen für den Anschluss entsprechender Schläuche aus dem Gehäuse heraus. Ansonsten ist das Gehäuse üblicherweise eben, so dass es sich leicht reinigen und desinfizieren lässt. Das Gehäuse nimmt entweder eine autonome Stromversorgung in sich auf und/oder ist mit einem Anschlusskabel für den elektrischen Anschluss der Pumpe an das Netz versehen.

Bei einem Pumpengehäuse mit achteckiger Grundfläche kann der Statusindikator beispielsweise auf zwei benachbarten Seitenflächen angeordnet sein. Die Flächennormalen der benachbarten Seitenflächen stehen jeweils in einem Winkel von 45° zueinander. Bei einem Pumpengehäuse mit viereckiger Grundfläche und stehen die Flächennormalen benachbarter Seitenflächen jeweils in einem Winkel von 90° zueinander und der Statusindikator kann beispielsweise auf zwei unterschiedlichen, benachbarten oder gegenüberliegenden Seitenflächen angeordnet sein. Der Statusindikator muss dabei nicht notwendigerweise auf einer Seitenfläche platziert sein, sondern kann beispielsweise auch auf der Oberseite des Pumpengehäuses angeordnet sein.

Gemäß einer zweiten Variante der Erfindung, weist die erfindungsgemäße Saugpumpe einen oder mehrere Standfüße zur Auflage auf einer Auflagefläche auf. Durch diese Standfüße wird ein definierter Abstand zwischen der Unterseite der Saugpumpe und der Auflagefläche hergestellt. In dieser Variante ist die Saugpumpe dadurch gekennzeichnet, dass der Statusindikator auf der Unterseite der Saugpumpe angeordnet ist, so dass das Lichtsignal in Richtung der Auflagefläche abgegeben wird. Das Lichtsignal wird in diesem Fall von der Auflagefläche reflektiert und durch den Spalt zwischen der Unterseite der Saugpumpe und der Auflagefläche in die Umgebung der Saugpumpe geleitet. Auf diese Weise wird das Lichtsignal in einem breiten Blickwinkelbereich abgestrahlt und ist somit auch aus größerer Entfernung gut zu erkennen. Dadurch kann der Betriebszustand der Saugpumpe auf einfache Weise überprüft werden. In einer bevorzugten Ausführungsform ist der Statusindikator so auf der Unterseite der Saugpumpe angeordnet, dass das von der Auflagefläche reflektierte Licht in einem Blickwinkelbereich von mindestens 45°, bevorzugt mindestens 90°, besonders bevorzugt 180°, am meisten bevorzugt 360° in horizontaler Ebene um die Saugpumpe herum abgestrahlt wird.

Nach einer weiteren alternativen Ausgestaltung der vorliegenden Erfindung nimmt der Statusindikator mindestens 5%, bevorzugt mindestens 10%, weiter bevorzugt mindestens 20%, besonders bevorzugt mindestens 30% oder mehr bis zu 100% der Oberfläche der Saugpumpe ein. Als Oberfläche ist dabei die Gesamtoberfläche der Saugpumpe zu verstehen, also auch diejenigen Flächenanteile, die beim üblichen Betrieb nicht sichtbar sind, da sie die Unterseite bilden, auf der die Saugpumpe steht bzw. von der Standfüße abragen, so dass die Unterfläche der Standfläche gegenüberliegend vorgesehen ist. Die Alternative gibt dementsprechend einen Statusindikator an, der relativ großflächig auf der Außenseite der Saugpumpe vorgesehen ist, was die Sichtbarkeit des oder der von dem Statusindikator abgegebenen Statussignale erhöht. Dabei kann der Statusindikator nur auf einer von außen sichtbaren Seitenfläche der Pumpe vorgesehen sein, beispielsweise allein einer der Seitenflächen oder der Oberfläche bei einer quader- oder kastenförmigen Ausgestaltung des Gehäuses.

In einer bevorzugten Ausführungsform weist die Saugpumpe ein Pumpengehäuse auf, welches eine vordere Fläche, zwei daran anschließende Seitenflächen und eine obere Fläche umfasst. Als vordere Fläche wird hierbei die dem Nutzer zugewandte Seite des Pumpengehäuses bezeichnet. Die Seitenflächen schließen sich seitlich an die vordere Fläche an, die obere Fläche schließt das Pumpengehäuse nach oben hin ab. Hierbei ist der Statusindikator auf wenigstens zwei Punkten von mindestens zwei der genannten Flächen angeordnet, deren Flächennormalen in einem Winkel von mindestens 5° oder mindestens einem ganzzahligen Vielfachen von 5° aber nicht mehr als 180° zueinanderstehen. Bevorzugt stehen die Flächennormalen in einem Winkel von mindestens 45°, besonders bevorzugt mindestens 90° zueinander.

Eine besonders gute Sichtbarkeit des Statusindikators wird dadurch erzielt, dass der Statusindikator sowohl auf mindestens drei der genannten Flächen, beispielsweise der vorderen Fläche und beiden Seitenflächen angeordnet ist.

Die genannten Flächen selbst sind vorzugsweise eben, können jedoch zumindest teilweise gerundet sein.

In einer Ausführungsform ist das beschriebene Gehäuse quader- oder kastenförmig, so dass die vordere Fläche, die daran anschließenden Seitenflächen und die obere Fläche jeweils im rechten Winkel zueinanderstehen. In einer Variante dieser Ausführungsform kann die obere Fläche auch schräg verlaufen und beispielsweise in einem Winkel von mehr oder weniger als 90° zu der vorderen oder einer der Seitenflächen stehen.

Die zwischen den genannten Flächen verlaufenden Kanten müssen nicht notwendigerweise rechtwinklig sein, sondern können auch abgeschrägt oder abgerundet ausgestaltet sein. Die Kanten können ganz oder teilweise abgeschrägt sein, so dass zwischen zwei oder mehr der oben genannten Flächen zusätzliche Schrägflächen ergeben, die wie die Kanten mit einem Statusindikator oder einem Teil davon versehen sein können.

Das Pumpengehäuse kann auch eine zumindest teilweise runde äußere Oberfläche aufweisen. Beispielsweise kann das Pumpengehäuse zylinderförmig, kegelförmig, kugel- oder halbkugelförmig ausgestaltet sein. Auch in diesem Fall ist der Statusindikator auf mindestens zwei Punkten der äußeren Oberfläche angebracht, deren Flächennormalen in einem Winkel von mindestens 5°, bevorzugt mindestens einem ganzzahligen Vielfachen von 5°, besonders bevorzugt mindestens 45°, weiter bevorzugt mindestens 90°, am meisten bevorzugt in einem Winkel von 180° zueinanderstehen.

Eine besonders gute Sichtbarkeit des Lichtsignales wird dadurch erzielt, dass der Statusindikator auf den genannten Flächen jeweils in Form von Lichtbändern ausgebildet ist, die sich vorzugsweise jeweils zwischen zwei Kanten der Flächen erstrecken. Die Lichtbänder erstrecken sich dabei zumindest abschnittsweise auf den genannten Flächen und/oder den Kanten. Vorzugsweise erstrecken sich die Lichtbänder jeweils durchgehend zwischen zwei Kanten der genannten Flächen. Besonders bevorzugt erstrecken sich die Lichtbänder durchgehend über mindestens zwei der genannten Flächen. Die Lichtbänder können dabei beispielsweise in gerader Form horizontal, vertikal oder schräg verlaufen. Alternativ können die Lichtbänder einer geschwungenen Kontur, beispielsweise einer Wellenlinie, folgen. Vorzugsweise hat der Statusindikator die Form eines einzigen Lichtbandes, das sich durchgängig über die vordere Fläche und die beiden Seitenflächen erstreckt. Dieses Lichtband kann beispielsweise in gerader Form horizontal oder entlang einer Wellenlinie verlaufen. Vorzugsweise bildet das Lichtband eine in sich geschlossene Kurve.

In einer alternativen Ausführungsform ist der Statusindikator auf den genannten Flächen jeweils in Form von einem oder mehreren diskreten Leuchtelementen ausgebildet. Vorzugsweise handelt es sich hierbei um flächige, nicht punktförmige Leuchtelemente. Die Leuchtelemente umfassen vorzugsweise ein Diffusorelement, durch das das Lichtsignal hindurchtritt, so dass die Lichtintensität über die gesamte durchleuchtete Oberfläche der Leuchtelemente homogen verteilt ist. Die diskreten Leuchtelemente können beispielsweise kreisförmig, ellipsoidal oder rechteckig geformt sein. Die diskreten Leuchtelemente können auch in Form von in sich geschlossenen Lichtbändern, also beispielsweise ringförmig, ausgeführt sein. Vorzugsweise befinden sich auf den beiden Seitenflächen des Pumpengehäuses jeweils mindestens zwei diskrete Leuchtelemente.

Der Statusindikator ist vorzugsweise großflächig ausgebildet und nimmt vorzugsweise mindestens 5 %, bevorzugt mindestens 10 %, besonders bevorzugt mindestens 20 %, am meisten bevorzugt mindestens 30 % der äußeren Oberfläche der Saugpumpe ein. Obwohl denkbar ist, dass die gesamte äußere Oberfläche der Saugpumpe vom Statusindikator abgedeckt wird, nimmt der Statusindikator praktischerweise nicht mehr als 90 %, bevorzugt nicht mehr als 70 %, besonders bevorzugt nicht mehr als 50 % der äußeren Oberfläche ein.

In Fall eines runden Gehäuses, kann der Statusindikator beispielsweise in Form von mehreren diskreten Leuchtelementen über den Außenumfang der runden äußeren Oberfläche verteilt sein. Vorzugsweise ist der Statusindikator hierbei jedoch als durchgängiges Lichtband ausgeführt, welches zumindest entlang eines Teils des Außenumfangs des Gehäuses über einen Winkelbereich von 5° oder mehr, vorzugsweise 45° oder mehr verläuft. Vorzugsweise verläuft das Lichtband vollumfänglich.

Das Pumpengehäuse selbst kann einstückig ausgebildet sein, oder aus mehreren miteinander verbundenen Gehäuseteilen bestehen. In einer bevorzugten Ausführung umfasst das Pumpengehäuse zwei Bauteile, die jeweils eine vordere Fläche und zwei daran anschließende Seitenflächen aufweisen und die entlang einer horizontal verlaufenden Naht miteinander verbunden sind. Hier kann die Naht selbst aus einem lichtdurchlässigen Material gefertigt sein, welches von innen beleuchtet wird und somit den optischen Statusindikator bildet.

In einer weiteren Ausführungsform weist die Saugpumpe einen Behälter zur Aufnahme einer Flüssigkeit auf. Insbesondere handelt es sich dabei um einen Fluidsammelbehälter zur Aufnahme der abzupumpenden Körperflüssigkeit. Dieser Behälter ist vorzugsweise abnehmbar mit der Saugpumpe verbunden. Insbesondere stellt der Behälter vorzugsweise ein Verbrauchsmaterial dar und wird nach Benutzung gegen einen neuen Behälter ausgetauscht. Vorzugsweise bildet die Außenwand des Behälters im montierten Zustand einen Teil der äußeren Oberfläche der Saugpumpe. Der Behälter kann beispielsweise eine hintere Fläche und zwei daran anschließende Seitenflächen ausbilden, die zusammen einen Teil der äußeren Oberfläche der Saugpumpe bilden.

In einer bevorzugten Ausführungsform ist der Statusindikator auf der äußeren Oberfläche des Behälters angeordnet. Dabei kann der Statusindikator beispielsweise wie oben beschrieben in Form eines oder mehrerer Lichtbänder oder diskreter Leuchtelemente ausgeführt sein.

Besonders bevorzugt ist es indes, wenn die Außenwand des Behälters oder ein Teil davon aus einem lichtdurchlässigen Material bestehen, welches von innen beleuchtet wird. Der Behälter wird dabei erfindungsgemäß so beleuchtet, dass das Lichtsignal auf zumindest zwei Punkten der äußeren Oberfläche des Behälters nach außen tritt, deren Flächennormalen in dem oben genannten Winkel zueinanderstehen. Die Außenwand des Behälters oder Teile davon können beispielsweise aus einem Lichtleiter gefertigt sein, in das von einer innerhalb der Saugpumpe angeordneten Lichtquelle erzeugte Lichtsignal eingespeist und über Lichtumlenkstrukturen im Lichtleiter nach Außen umgeleitet wird. Der Behälter kann auch aus einem transparenten Kunststoff bestehen, der von innen und/oder außen beleuchtet wird, wobei die Lichtquelle in der Regel in oder an dem Gehäuse der Pumpe untergebracht ist. Dies ist insbesondere bei Einweg-Behältern aus Kunststoff von Vorteil, die nach Benutzung entsorgt werden.

In einer weiteren Ausführungsform umfasst die Saugpumpe eine Durchflussleitung zur Verbindung der Saugpumpe mit einer Fluidsammelvorrichtung. Über die Durchflussleitung kann Flüssigkeit vom Patienten über die Fluidsammelvorrichtung abgeleitet werden und beispielsweise in dem oben beschriebenen Behälter gesammelt werden. Bei der Fluidsammelvorrichtung handelt es sich beispielsweise um eine eingangs erwähnte Brusthaube oder einen Vakuumverband. Die Durchflussleitung umfasst vorzugsweise eine Kupplung, mit der sie an eine Fluidsammelvorrichtung oder ein weiterführendes Schlauchstück angeschlossen werden kann. Die Kupplung ist beispielsweise als Luer- oder Luer-Lock-Verbindung ausgeführt. Die Durchflussleitung kann beispielsweise einen kreisförmigen oder rechteckigen Querschnitt aufweisen, wobei ein kreisrunder Querschnitt bevorzugt ist.

In einer bevorzugten Ausführungsform ist der Statusindikator auf der äußeren Oberfläche der Durchflussleitung angebracht, so dass er auf zumindest zwei Punkten dieser äußeren Oberfläche angeordnet ist, deren Flächennormalen in einem Winkel von mindestens 5°, bevorzugt mindestens einem ganzzahligen Vielfachen von 5°, besonders bevorzugt mindestens 45°, am meisten bevorzugt mindestens 90° zueinanderstehen. Dabei kann der Statusindikator beispielsweise wie oben beschrieben in Form eines oder mehrerer Lichtbänder oder diskreter Leuchtelemente ausgeführt sein. Das Lichtband kann beispielsweise um die Durchflussleitung gewickelt sein. Besonders bevorzugt ist, wenn der Statusindikator als Lichtband ausgeführt ist, welches den Außenumfang der Durchflussleitung vollumfänglich umschließt. Besonders bevorzugt ist es außerdem, wenn die Außenwand der Durchflussleitung oder ein Teil davon aus einem lichtdurchlässigen Material bestehen, welches von innen beleuchtet wird. Die Außenwand der Durchflussleitung oder Teile davon können beispielsweise aus einem Lichtleiter gefertigt sein, in das von einer innerhalb der Saugpumpe angeordneten Lichtquelle erzeugte Lichtsignal eingespeist und über Lichtumlenkstrukturen im Lichtleiter nach Außen umgeleitet wird. Beispielsweise kann die Durchflussleitung aus einem transparenten Kunststoff bestehen, der als Lichtleiter dient und von innen und/oder außen beleuchtet wird. Auch hier ist die Lichtquelle in der Regel in oder an dem Gehäuse der Pumpe untergebracht.

Das Lichtsignal wird durch eine oder mehrere Lichtquellen erzeugt. Beispielsweise kann die Saugpumpe eine einzelne Lichtquelle aufweisen, deren Licht über einen oder mehrere Lichtleiter an die betreffenden Punkte der äußeren Oberfläche der Saugpumpe weitergeleitet wird und dort durch ein lichtdurchlässiges Element nach Außen tritt. In diesem Fall wird der Statusindikator durch die auf der Oberfläche der Saugpumpe angeordneten lichtdurchlässigen Elemente gebildet. Eine LED ist in diesem Fall eine besonders geeignete Lichtquelle.

In einer Ausführungsform besteht ein Teil der äußeren Oberfläche der Saugpumpe aus einem lichtdurchlässigen Material, in welches das von einer im Innern der Saugpumpe angeordneten Lichtquelle erzeugte Lichtsignal eingekoppelt wird. Über geeignete Lichtumlenkstrukturen innerhalb des lichtdurchlässigen Materials wird das Lichtsignal dann nach Außen gelenkt.

Die Saugpumpe kann auch mehrere Lichtquellen aufweisen, die nebeneinander auf den betreffenden Punkten der äußeren Oberfläche angeordnet sind und zusammen den Statusindikator bilden. Hierbei können beispielsweise punktförmige LEDs eingesetzt werden. Diese können beispielsweise in Reihe oder in einem Raster angeordnet sein.

Eine weitere geeignete Lichtquelle sind OLEDs, die den besonderen Vorteil haben, in Dünnschichttechnik in flächiger Form auf die betreffenden Punkte der äußeren Oberfläche aufgebracht zu werden. In einer bevorzugten Ausführungsform umfasst der Statusindikator ein oder mehrere OLEDs, die auf der äußeren Oberfläche der Saugpumpe angeordnet sind.

Geeignete lichtleitenden Elemente bzw. Lichtquellen zur Herstellung des erfindungsgemäßen Statusindikators sind aus dem Stand der Technik bekannt und beispielsweise in EP 3228931 A1, EP 3326863 A1, WO 2014/033686 A2, WO 2015/116743 A1, DE 10 2009 051 234 A1 und US 6728464 A1 beschrieben.

Vorzugsweise wird das Lichtsignal durch ein Diffusorelement geleitet, so dass die Lichtintensität über gesamte Oberfläche des Statusindikators homogen verteilt ist. Dadurch wird eine gleichmäßige Helligkeit des Lichtsignals erzeugt, was die Wahrnehmung des Lichtsignals aus unterschiedlichen Blickwinkeln erleichtert. In einer alternative Ausführungsform ist die Lichtintensität über die Oberfläche des Statusindikators inhomogen verteilt. Beispielsweise kann das Lichtsignal in diesem Fall ein Streifenmuster bilden.

In einer Ausführungsform umfasst die Saugpumpe eine Vorrichtung zur Messung der Umgebungshelligkeit und zur Anpassung der Intensität des Lichtsignals an die Umgebungshelligkeit. Vorzugsweise umfasst die Vorrichtung einen Helligkeitssensor, dessen Sensorsignal von einer Steuereinheit der Saugpumpe zur Steuerung der Intensität des Lichtsignals in Abhängigkeit von der ermittelten Umgebungshelligkeit verarbeitet wird. Auf diese Weise kann die Helligkeit des Statusindikators bei Nacht reduziert werden, um Energie zu sparen und die Nachtruhe des Patienten nicht zu stören. Gleichzeitig kann die Helligkeit bei Tag automatisch erhöht werden, um eine gute Erkennbarkeit des Statusindikators zu gewährleisten.

Das vom Statusindikator angezeigte Lichtsignal wird in Abhängigkeit vom Betriebszustand der Saugpumpe erzeugt oder angepasst. Der Betriebszustand wird vorzugsweise anhand von einem oder mehreren Betriebsparametern bestimmt. Beispielsweise handelt es sich bei diesen Parametern um den Füllstand des oben beschriebenen Behälters zur Aufnahme der Körperflüssigkeit, um den in der oben beschriebenen Durchflussleitung bzw. einer daran angeschlossenen Schlauchverbindung oder Flüssigkeitssammelvorrichtung anliegenden Unterdruck, um die in der Durchflussleitung oder einer daran angeschlossenen Schlauchverbindung gemessene Durchflussrate oder bei einer mittels Batterie betriebenen Saugpumpe um den Ladestand der Batterie. Durch Vergleich des gemessenen Betriebsparameters mit einem vorgegebenen Referenzwert kann festgestellt werden, ob eine Betriebsstörung der Saugpumpe und/oder eine Abweichung vom vorgegebenen Prozessverlauf vorliegt.

Eine Betriebsstörung liegt beispielsweise vor, wenn der Füllstand des Behälters ein vorgegebenes Maximum überschreitet, wenn das Pumpenaggregat nicht mit der vorgegebenen Pumpleistung arbeitet oder wenn der Ladestand einer gegebenenfalls vorhandenen Batterie ein vorgegebenes Minimum unterschreitet. Eine Abweichung vom vorgegebenen Prozessverlauf besteht z.B. wenn es aufgrund eines Lecks in der Durchflussleitung oder einer daran angeschlossenen Schlauchverbindung oder einer Undichtigkeit eines Vakuumverbandes zu einer veränderten Durchflussrate kommt.

Das Lichtsignal wird vorzugsweise automatisch von einer in der Saugpumpe angeordneten Steuereinrichtung ausgelöst oder angepasst. Vorzugsweise wird das Lichtsignal beim Vorliegen einer Betriebsstörung und/oder einer Abweichung vom vorgegebenen Prozessverlauf ausgelöst oder angepasst, beispielsweise, wenn der Maximalfüllstand des Behälters überschritten wird, die Durchflussrate in der Durchflussleitung oder einer daran angeschlossenen Schlauchverbindung, oder der in der Durchflussleitung anliegende Unterdruck von einem vorgegebenen Referenzwert abweicht bzw. vorgegebene Grenzwerte über- oder unterschreitet.

Die Intensität des Lichtsignals kann zeitlich konstant sein oder periodisch variieren (blinken). Vorzugsweise kann die Steuervorrichtung je nach Schwere der Betriebsstörung zwischen diesen beiden Alternativen wechseln. Beispielsweise kann ein konstantes Lichtsignal erzeugt werden, wenn sich der Füllstand des Behälters seinem Maximum nähert, und ein blickendes Lichtsignal, wenn der Füllstand das Maximum überschreitet. Auch die Frequenz des Blinkens kann erhöht werden, um eine besonders schwere Betriebsstörung anzuzeigen.

In einer Ausführungsform wird das Lichtsignal in unterschiedlichen Farben in Abhängigkeit von dem Betriebszustand erzeugt. So wird beispielsweise bei störungsfreiem Betrieb ein Lichtsignal in einer Farbe (beispielsweise grün) erzeugt und beim Vorliegen einer Betriebsstörung und/oder einer Abweichung vom vorgegebenen Prozessverlauf ein Lichtsignal in einer anderen Farbe (beispielsweise rot). Auf diese Weise kann das Betriebspersonal auf einfache Weise den Betriebszustand der Saugpumpe überprüfen.

Der Statusindikator kann über seine gesamte Fläche ein einheitliches Lichtsignal anzeigen, welches eine einzelne Statusinformation vermittelt. Beispielsweise zeigt der Statusindikator in diesem Fall an, ob eine Störung des Betriebszustands vorliegt oder nicht. Alternativ kann der Statusindikator auch in unterschiedliche Abschnitte aufgeteilt sein, die jeweils unterschiedliche Lichtsignale anzeigen. Auf diese Weise können unterschiedliche Statusinformationen vermittelt werden. Beispielsweise können hierdurch verschiedene Arten der Betriebsstörung voneinander unterschieden werden.

Die Stromversorgung der Saugpumpe kann über einen Netzanschluss oder eine interne Batterie erfolgen. Im Falle einer Batterie kann die Saugpumpe tragbar ausgestaltet sein und muss nicht unbedingt stationär betrieben werden. Vorzugsweise verfügt die Saugpumpe im Falle einer Batterie über einen Stromanschluss, über den die Batterie geladen werden kann.

Weitere bevorzugte Merkmale der Erfindung ergeben sich aus den abhängigen Ansprüchen und den im Folgenden beschriebenen Ausführungsformen.

### Beschreibung der Zeichnungen

Bevorzugte Ausführungsformen der Erfindung werden im Folgenden anhand von Zeichnungen beschrieben, die lediglich zur Erläuterung der Erfindung dienen und nicht einschränkend auszulegen sind. In den Zeichnungen zeigen:
- Figur 1:: Schematische Darstellung einer Saugpumpe gemäß einer ersten Ausführungsform.
- Figur 2:: Schematische Darstellung einer Saugpumpe mit schräger oberer Fläche.
- Figuren 3 und 4:: Schematische Darstellung einer Saugpumpe mit einem Statusindikator in Form eines durchgängigen Lichtbands auf der vorderen Fläche und den beiden Seitenflächen des Pumpengehäuses.
- Figur 5:: Schematische Darstellung einer Saugpumpe mit einem Statusindikator in Form eines Lichtbandes auf den beiden Seitenflächen des Pumpengehäuses.
- Figuren 6 und 7:: Schematische Darstellung einer Saugpumpe mit einem Statusindikator in Form von diskreten Leuchtelementen.
- Figur 8:: Schematische Darstellung einer Saugpumpe mit einem auf der äußeren Oberfläche der Durchflussleitung angebrachten Statusindikator.
- Figur 9:: Schematische Darstellung einer Saugpumpe gemäß der zweiten Variante der Erfindung mit einem auf der Unterseite der Saugpumpe angebrachten Statusindikator.
- Figuren 10 A und B:: Schematische Darstellungen zweier Saugpumpen gemäß der ersten Variante der Erfindung mit achteckiger Grundfläche bzw. runder Grundfläche.

### Beschreibung bevorzugter Ausführungsformen

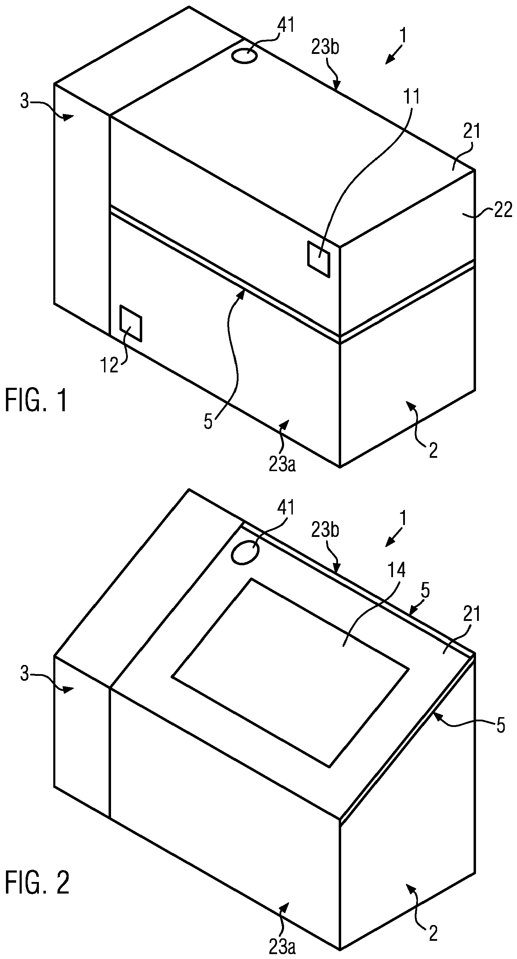

Figur 1 zeigt eine Saugpumpe 1 zur Wunddrainage gemäß der ersten Variante der Erfindung. Die Pumpe verfügt über einen Ein/Aus-Schalter 11 und einen Stromanschluss 12. Auf der Unterseite der Saugpumpe 1 können ein oder mehrere hier nicht gezeigte Standfüße 13 vorgesehen sein, um die Saugpumpe 1 auf einer Auflagefläche abzustellen.

Die Stromversorgung erfolgt in der gezeigten Saugpumpe 1 vorzugsweise über den Stromanschluss 12, der über ein Netzkabel dem Stromnetz verbunden werden kann. Alternativ oder ergänzend kann die Saugpumpe 1 jedoch auch eine Batterie zur Stromversorgung aufweisen. In diesem Fall kann die Saugpumpe 1 tragbar ausgestaltet sein und muss nicht unbedingt stationär betrieben werden. Die Batterie kann in diesem Fall vorzugsweise über den Stromanschluss 12 geladen werden.

Die Saugpumpe 1 besitzt ein kastenförmiges Pumpengehäuse 2, das eine obere Fläche 21, eine vordere Fläche 22 und zwei an die vordere Fläche 22 anschließende Seitenflächen 23a, 23b aufweist. Die (nicht eingezeichneten) Flächennormalen der vorderen Fläche 22, der oberen Fläche 21 und der Seitenflächen 23a, 23b stehen hierbei jeweils in einem Winkel 90° bzw. 180° zueinander. Das Pumpengehäuse 2 kann beispielsweise aus Kunststoff oder Aluminium gefertigt sein. Vorzugsweise ist das Pumpengehäuse aus Kunststoff gefertigt. Auf der oberen Fläche 21 kann auch ein Display zur Anzeige unterschiedlicher Betriebsparameter und/oder ein Bedienfeld zur Einstellung von Betriebsparametern vorgesehen sein.

Auf der Rückseite der Saugpumpe 1 ist ein Behälter 3 angebracht. Dieser dient zur Aufnahme der abgepumpten Körperflüssigkeit. Der Behälter 3 bildet mit seinen Außenwänden einen Teil der äußeren Oberfläche der Saugpumpe 1 und schließt bündig mit der oberen Fläche 21 und den beiden Seitenflächen 23a, 23b des Pumpengehäuses 5 ab. Der Behälter 3 ist vorteilhafterweise lösbar zum Austausch oder zum Entleeren mit der Saugpumpe 1 verbunden. Zu diesem Zweck verfügt die Saugpumpe 1 über einen Entriegelungsmechanismus. Vorteilhafterweise verfügt die Saugpumpe 1 über einen Füllstandsensor, mit dem die im Behälter 3 gesammelte Flüssigkeitsmenge laufend gemessen und das Messergebnis an eine Steuereinheit weitergeleitet werden kann.

Auf der Oberseite der Saugpumpe 1 ist eine in Figur 1 nicht gezeigte Durchflussleitung 4 vorgesehen. Diese hat vorteilhafterweise einen kreisrunden Querschnitt. Die Durchflussleitung 4 dient zur Verbindung der Saugpumpe 1 mit einer hier nicht gezeigten Flüssigkeitssammelvorrichtung, wie beispielsweise einem Vakuumverband. Die Durchflussleitung 4 ist innerhalb des Pumpengehäuses 2 mit einem hier nicht gezeigten Pumpenaggregat zur Erzeugung eines Unterdrucks und dem Behälter 3 verbunden. Über eine Schlauchaufnahmeöffnung 41 auf der Oberseite der Saugpumpe 1 wird die Durchflussleitung 4 aus dem Gehäuse hinausgeführt. An ihrem distalen Ende kann die Durchflussleitung 4 eine Kupplung aufweisen, die zum Anschluss an die Flüssigkeitssammelvorrichtung, vorzugsweise über eine weiterführende Schlauchleitung dient.

Ein optischer Statusindikator 5 ist in Form eines durchgängigen Lichtbandes auf der vorderen Fläche 22 und den beiden Seitenflächen 23a, 23b angeordnet. Dieses Lichtband reicht von der hinteren Kante der einen Seitenfläche 23a bis zur hinteren Kante der gegenüberliegenden Seitenfläche 23b.

Der Statusindikator 5 leuchtet beispielsweise auf, wenn eine Störung der Saugpumpe 1 vorliegt. Beispielsweise ist dies der Fall, wenn ein geeigneter Füllstandsensor eine Überschreitung des maximalen Füllstandes des Behälters 3, oder wenn ein geeigneter Drucksensor eine Abweichung des Unterdrucks innerhalb der Durchflussleitung 4 von vorher festgelegten Grenzwerten feststellt.

In einer vorteilhaften Ausgestaltung des Statusindikators 5 leuchtet dieser je nach Betriebszustand in unterschiedlichen Farben. Beispielsweise leuchtet der Statusindikator 1 in einer bestimmten Farbe, falls keine Störung vorliegt, und in einer anderen Farbe, falls eine Störung festgestellt wurde.

Der Statusindikator 5 ist vorteilhafterweise in das Material des Pumpengehäuses 5 integriert, beispielsweise indem ein Teil des Pumpengehäuses aus lichtdurchlässigem Material gefertigt ist, in das über einen Lichtleiter das von einer innenliegenden Lichtquelle, beispielsweise einer LED, erzeugte Lichtsignal eingekoppelt wird. Das Lichtsignal wird vorteilhafterweise so eingekoppelt, dass die Lichtintensität über die gesamte Länge des Statusindikators 5 homogen verteilt ist. Alternativ kann die Lichtintensität auch ungleichmäßig über die Länge des Statusindikators 5 verteilt sein. Zum Beispiel kann das Licht ein Bandenmuster aufweisen.

Die Saugpumpe 1 verfügt vorteilhafterweise über einen hier nicht gezeigten Helligkeitssensor zur Messung der Umgebungshelligkeit und eine daran angeschlossene Steuereinheit, welche die Helligkeit des Statusindikators 5 an die jeweilige Umgebungshelligkeit anpasst.

Die Figuren 2 bis 7 zeigen Varianten der in Figur 1 gezeigten Saugpumpe 1.

Gemäß Figur 2 verfügt die Saugpumpe 1 über ein Pumpengehäuse 2 mit schräg verlaufender oberer Fläche 21. Ein Statusindikator 5 ist in Form von Lichtbändern sowohl auf der oberen Fläche 21 als auch auf einer Seitenfläche 23a des Gehäuses angeordnet. Zusätzlich kann auf der oberen Fläche 21 ein Display 14 angeordnet sein. Über das Display 14 können weitere Informationen zum Betriebszustand der Saugpumpe 1 angezeigt werden. Das Display kann außerdem als berührungsempfindlicher Bildschirm ausgeführt sein, über den die Funktion der Saugpumpe 1 vom Nutzer gesteuert werden kann. In Abwandlung zu der in Figur 1 gezeigten Saugpumpe 1, ist der Behälter 3 nicht auf der Rückseite der Saugpumpe 1, sondern seitlich angeordnet.

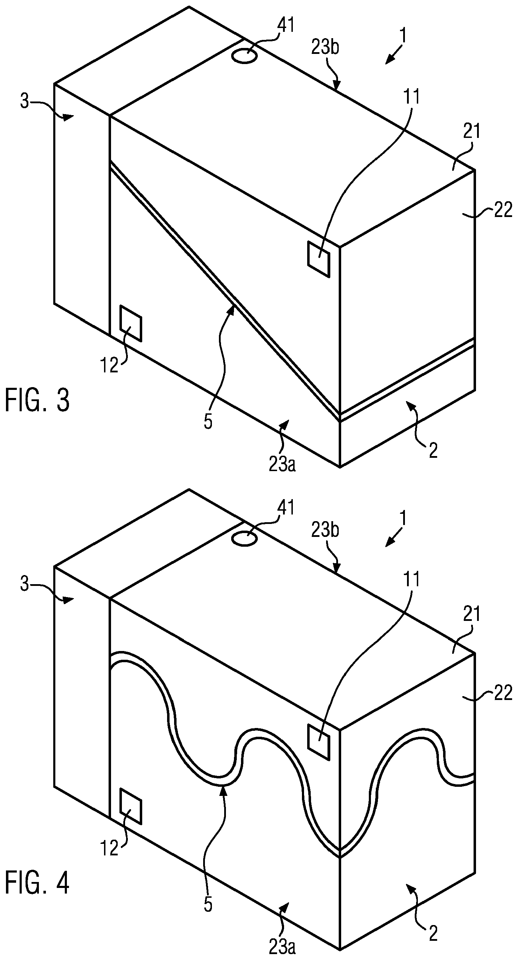

Die Figuren 3 und 4 zeigen einen Statusindikator 5, der sich in Form eines Lichtbandes über die vordere Fläche 22 und die beiden Seitenflächen 23a, 23b des Pumpengehäuses 2 erstreckt. In Figur 3 verläuft der Statusindikator 5 in schräger Linie über die beiden Seitenflächen 23a, 23b und in waagerechter Linie über die vordere Fläche 22. In Figur 4 verläuft der Statusindikator 5 entlang einer Wellenlinie.

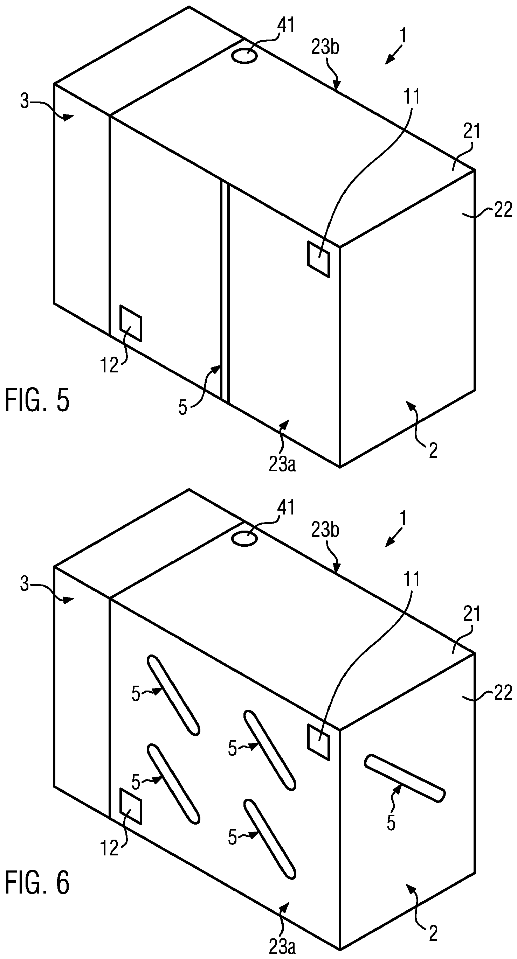

Die Figur 5 zeigt einen Statusindikator, der in Form von zwei durchgängigen, senkrechten Lichtbändern jeweils auf den beiden Seitenflächen 23a, 23b vorgesehen ist. Auf der vorderen Seite 22 ist hierbei kein Statusindikator angebracht.

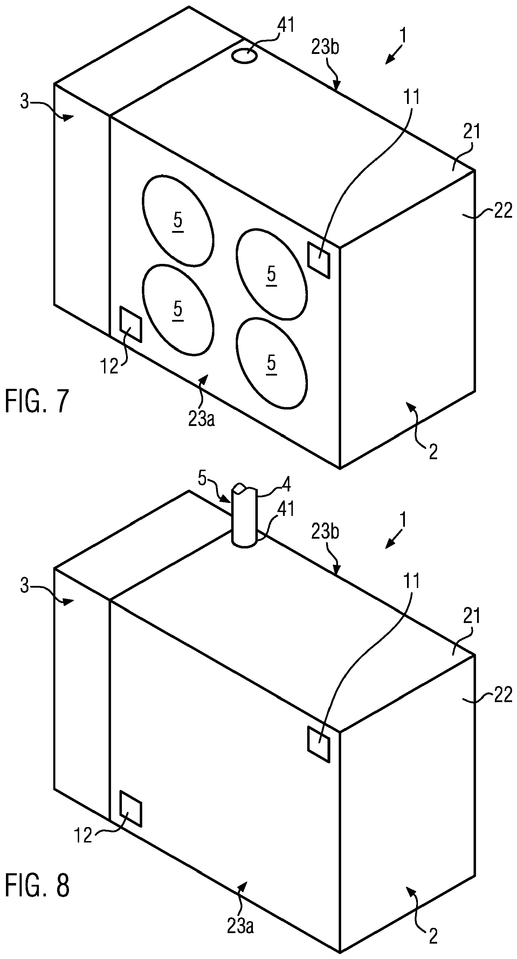

Die Figuren 6 und 7 zeigen einen Statusindikator 5 in Form einer Vielzahl von diskreten Leuchtelementen. In Figur 6 haben diese Leuchtelemente die Form von langgestreckten Rechtecken mit abgerundeten Kanten und sind sowohl an den beiden Seitenflächen 23a, 23b als auch auf der vorderen Fläche 22 angebracht. In Figur 7 sind die Leuchtelemente kreisförmig und nur auf den beiden Seitenflächen 23a, 23b angebracht.

Figur 8 zeigt eine weitere Ausführungsform der Erfindung, bei welcher die Durchflussleitung 4 aus einem lichtdurchlässigen Material gefertigt ist, welches von innen beleuchtet wird. Die Durchflussleitung 4 hat einen kreisrunden Querschnitt. Das von einer hier nicht gezeigten, im Innern der Saugpumpe 1 angeordneten Lichtquelle erzeugte Lichtsignal wird über Lichtleiter in das lichtdurchlässige Material eingespeist. Das lichtdurchlässige Material kann sich über die gesamte Länge der Durchflussleitung 4 erstrecken, oder nur ein Teil der Durchflussleitung 4, beispielsweise der Abschnitt zwischen der Schlauchaufnahmeöffnung 41 und der Kupplung 42, kann aus dem lichtdurchlässigen Material gefertigt sein. Auf diese Weise dient zumindest ein Teil der äußeren Oberfläche der Durchflussleitung 4 über den gesamten Außenumfang als optischer Statusindikator 5.

Figur 9 zeigt eine Saugpumpe 1 gemäß der zweiten Variante der Erfindung. Die Saugpumpe umfasst ein Gehäuse 2, einen Behälter 3, eine Durchflussleitung 4, einen An/Aus-Schalter 11 und einen Stromanschluss 12 wie bereits für die Saugpumpe von Figur 1 beschrieben. Die Saugpumpe 1 verfügt zusätzlich an ihrer Unterseite über Standfüße 13, mit der die Saugpumpe 1 auf einer Auflagefläche abgestellt werden kann. Die Standfüße 13 haben den Effekt, dass zwischen Auflagefläche und der Unterseite der Saugpumpe 1 ein definierter Abstand hergestellt wird. Ein optischer Statusindikator 5 ist auf der Unterseite der Saugpumpe 1 angebracht, so dass das vom Statusindikator 5 angezeigte Lichtsignal in Richtung der Auflagefläche abgegeben wird. Das in der Zeichnung durch die gestrichelten Pfeile dargestellte Lichtsignal wird von der Auflagefläche reflektiert und durch den Spalt zwischen Saugpumpe 1 und Auflagefläche nach Außen geleitet.

### Bezugszeichenliste

- 1: Saugpumpe
- 11: Ein/Aus-Schalter
- 12: Stromanschluss
- 13: Standfuß
- 14: Display
- 2: Pumpengehäuse
- 21: Obere Fläche
- 22: Vordere Fläche
- 23a, 23b: Seitenflächen
- 3: Behälter
- 4: Durchflussleitung
- 41: Schlauchaufnahmeöffnung
- 5: Statusindikator
- 6a-d: Flächennormalen

## Patentansprüche

1. Saugpumpe (1) zum Absaugen von Körperflüssigkeit mit einem optischen Statusindikator (5) zur Anzeige eines Lichtsignals in Abhängigkeit vom Betriebszustand der Saugpumpe (1), **dadurch gekennzeichnet, dass** der optische Statusindikator (5) auf mindestens zwei Punkten der äußeren Oberfläche der Saugpumpe (1) angeordnet ist, deren Flächennormalen in einem Winkel von mindestens 5° zueinanderstehen.

2. Saugpumpe (1) zum Absaugen von Körperflüssigkeit mit einem optischen Statusindikator (5) zur Anzeige eines Lichtsignals in Abhängigkeit vom Betriebszustand der Saugpumpe (1), wobei die Saugpumpe (1) auf ihrer Unterseite mindestens einen Standfuß (13) zur Auflage auf einer Auflagefläche aufweist, **dadurch gekennzeichnet, dass** der Statusindikator (5) auf der Unterseite der Saugpumpe (1) angeordnet ist, so dass das Lichtsignal in Richtung der Auflagefläche abgegeben wird.

3. Saugpumpe (1) zum Absaugen von Körperflüssigkeit mit einem optischen Statusindikator (5) zur Anzeige eines Lichtsignals in Abhängigkeit von Betriebszustand der Saugpumpe (1), **dadurch gekennzeichnet, dass** der Statusindikator (5) mindestens 5% der Oberfläche der Saugpumpe (1) einnimmt.

4. Saugpumpe (1) nach einem der Ansprüche 1 bis 3, wobei die Saugpumpe (1) ein Pumpengehäuse (2) aufweist, das eine obere Fläche (21), eine vordere Fläche (22), und zwei jeweils daran anschließende Seitenflächen (23) umfasst, und der Statusindikator (5) auf zumindest zwei Punkten von zumindest zwei der genannten Flächen (21, 22, 23a, 23b) angeordnet ist, deren Flächennormalen in einem Winkel von mindestens 5° zueinanderstehen.

5. Saugpumpe (1) nach Anspruch 4, wobei der Statusindikator (5) zumindest auf der vorderen Fläche (22) und den beiden Seitenflächen (23a, 23b) des Pumpengehäuses (2) angeordnet ist.

6. Saugpumpe (1) nach einem der Ansprüche 4 bis 5, wobei der Statusindikator (5) auf zumindest zwei der genannten Flächen (21, 22, 23a, 23b) jeweils als durchgängiges oder in Abschnitte unterteiltes Lichtband ausgeführt ist.

7. Saugpumpe (1) nach einem der Ansprüche 4 bis 6, wobei der Statusindikator (5) als ein durchgängiges oder in Abschnitte unterteiltes Lichtband ausgeführt ist, das sich zumindest auf der vorderen Fläche (22) und den beiden Seitenflächen (23a, 23b) des Pumpengehäuses (2) erstreckt.

8. Saugpumpe (1) nach einem der Ansprüche 3 bis 5, wobei der Statusindikator (5) auf zumindest zwei der genannten Flächen (21, 22, 23a, 23b) in Form von jeweils einem oder mehreren diskreten Leuchtelementen ausgeführt ist.

9. Saugpumpe (1) nach einem der Ansprüche 1 bis 8, wobei der Statusindikator in Form von wenigstens einem Lichtband ausgeführt ist, das eine in sich geschlossene Kurve bildet.

10. Saugpumpe (1) nach einem der Ansprüche 1 bis 9, wobei die Saugpumpe (1) einen Behälter (3) zur Aufnahme einer Flüssigkeit aufweist und der Statusindikator (5) auf zumindest zwei Punkten der äußeren Oberfläche des Behälters (3) angeordnet ist, deren Flächennormalen in einem Winkel von mindestens 5° zueinanderstehen.

11. Saugpumpe (1) nach einem der Ansprüche 1 bis 10, wobei die Saugpumpe (1) eine Durchflussleitung (4) zur Verbindung der Saugpumpe (1) mit einer Fluidsammelvorrichtung aufweist, und der Statusindikator (5) auf zumindest zwei Punkten der äußeren Oberfläche der Durchflussleitung (4) angeordnet ist, deren Flächennormalen in einem Winkel von zumindest 5° zueinanderstehen.

12. Saugpumpe (1) nach einem der Ansprüche 1 bis 11, wobei die Saugpumpe (1) zumindest eine Lichtquelle zur Erzeugung des Lichtsignals aufweist, wobei das Lichtsignal durch einen oder mehrere Lichtleiter an den Statusindikator (5) weitergeleitet wird.

13. Saugpumpe (1) nach einem der Ansprüche 1 bis 12, wobei der Statusindikator (5) ein oder mehrere OLEDs umfasst, die auf der äußeren Oberfläche der Saugpumpe angeordnet sind.

14. Saugpumpe (1) nach einem der Ansprüche 1 bis 13, wobei die Saugpumpe eine Vorrichtung zur Messung der Umgebungshelligkeit und zur Anpassung der Intensität des Lichtsignals an die Umgebungshelligkeit aufweist.

15. Saugpumpe (1) nach einem der Ansprüche 1 bis 14, wobei das Lichtsignal bei Vorliegen einer Betriebsstörung der Saugpumpe und/oder einer Abweichung vom vorgegebenen Prozessverlauf erzeugt oder angepasst wird.
